# EUROPEAN PATENT APPLICATION

(11) **EP 4 796 091 A1**
(43) Date of publication of application: **26.08.2026**
(21) Application number: 25195006.9
(22) Date of filing: 08.08.2025
(51) Int. Cl.: A61F 13/514

(54) **ABSORBENT ARTICLES HAVING LOW BASIS WEIGHT BACKSHEET**

(30) Priority: 21.02.2025 EP 25159431
(71) Applicant: Ontex BV, 9255 Buggenhout (BE); Ontex Group NV, 9320 Erembodegem (BE)
(72) Inventor: Lambertz, Christina, 56566 Neuwied (DE); Jansen, Bart, 2640 Mortsel (BE)
(74) Representative: Macchetta, Andrea

(57) **Abstract**

An absorbent article comprising a liquid permeable topsheet; an absorbent core; and a substantially liquid impermeable backsheet; wherein the absorbent core is sandwiched between the said topsheet and said backsheet; and wherein said backsheet is breathable, whereint said backsheet is made of a composite material, said composite material comprising, preferably consisting essentially of, even more preferably consisting of, a film and at least a layer of nonwoven fabric, wherein the film is positioned on a skin-facing surface of the backsheet and the nonwoven fabric is positioned on a garment facing surface of the backsheet, and wherein the composite material is substantially free of an adhesive layer between said film and said nonwoven, preferably wherein the film and nonwoven are substantially co-formed.

## Description

### TECHNICAL FIELD

The present disclosure pertains to the technical field of absorbent articles, and more in particular to disposable articles for personal hygiene (such as baby or adult diapers, pants or incontinence briefs or pads, as well as feminine hygiene articles such as liners). Most preferred absorbent articles herein are diapers and/or pants, preferably baby diapers and/or pants.

### BACKGROUND

In recent years, the demand for eco-friendly absorbent articles has grown significantly, driving innovation towards the use of more sustainable materials and reducing reliance on chemicals. Compared to traditional disposable absorbent articles, modern alternatives increasingly prioritize environmental responsibility without compromising performance.

Whilst efforts have been done to reduce the basis weight of polyethylene-based backsheet films, such as exemplified in EP3142858 B1; such films however still present processing challenges when reducing the basis weight of the film below 11-12 gsm, let alone reducing basis weight of a film-nonwoven composite containing the same.

Thus, there remains a need for improved absorbent article solutions that balance sustainability with the practical leakage-prevention benefits of traditional impermeable backsheets used therein while providing a tactile experience of enhanced softness whilst preferably attaining the lowest possible grammage.

### SUMMARY

In a first aspect the disclosure relates to an absorbent article comprising a liquid permeable topsheet; and absorbent core; and a substantially liquid impermeable backsheet; wherein the absorbent core is sandwiched between the said topsheet and said backsheet; and wherein said backsheet is breathable, whereint said backsheet is made of a composite material, said composite material comprising, preferably consisting essentially of, even more preferably consisting of, a film and at least a layer of nonwoven fabric, wherein the film is positioned on a skin-facing surface of the backsheet and the nonwoven fabric is positioned on a garment facing surface of the backsheet, and wherein the composite material is substantially free of an adhesive layer between said film and said nonwoven, preferably wherein the film and nonwoven are substantially co-formed..

In a highly preferred aspect, the film has a basis weight of less than 10 gsm, preferably less than 8 gsm, more preferably from 1 gsm to 5 gsm, even more preferably from 2gsm to 4 gsm, even more preferably about 3 gsm.

In a further highly preferred aspect, the film and the nonwoven a co-formed by extrusion.

In a further aspect the disclosure relates to a method for manufacturing an absorbent article, the method comprising the, preferably sequential, steps of:
i. co-forming, preferably co-extruding, a film and nonwoven to form a composite material web;
ii. providing a liquid permeable nonwoven web;
iii. providing an absorbent core; and
iv. laminating the composite material web to at lest one of the liquid permeable nonwoven web and the absorbent core such that the absorbent core is sandwiched therebetween.

### BRIEF DESCRIPTION OF THE FIGURES

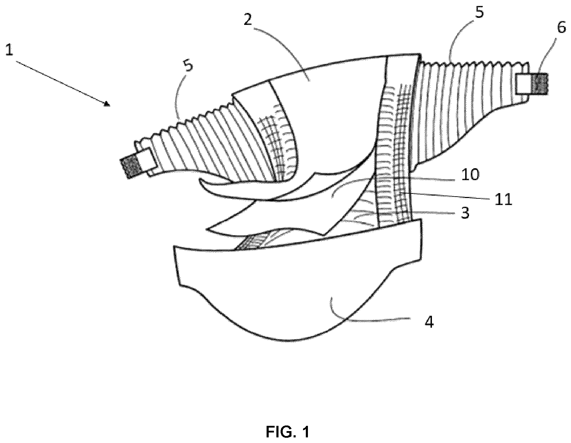

FIG. 1 is a perspective, partly exploded, view of an absorbent article according to an embodiment of the present disclosure.

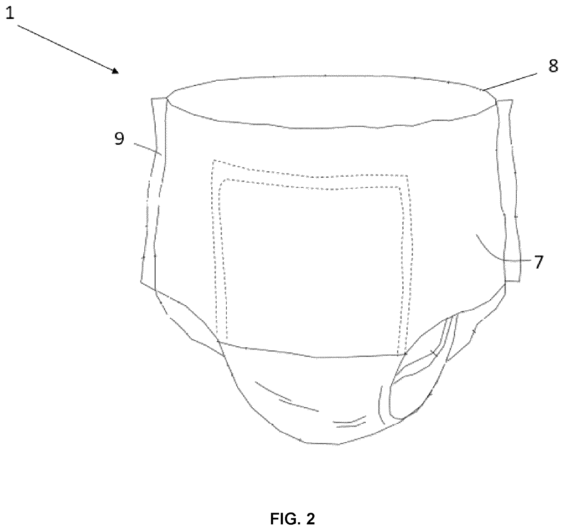

FIG. 2 is a perspective view of an article according to an embodiment of the present disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

As used herein "substantially free of adhesive" means less than 20gsm, preferably less than 15gsm, even more preferably less than 10gsm, even more preferable less than 5gsm, most preferably about 0gsm, of adhesive.

"WVTR" means "water vapor transmission rate," and is a measure of film breathability. WVTR is expressed in units of g H2O/24 hours/m2 or equivalent units thereof (e.g. g/m2/24h), and may be measured according to ISO 15106 (15106-2) and the equipment Lyssy L80-5000 may be used. Alternatively, the WVTR may be measured using Test ASTM 1249 and equipment Ametek Mocon.

As used herein "essentially free" (of polyethylene) means a material, component, element, or product that contains polyethylene in quantities so minimal that its presence is considered insignificant in terms of both mass and functional impact. The polyethylene content is hence typically below detection limits using standard analytical techniques, and/or it constitutes a negligible fraction of the overall composition, having no meaningful influence on the material's physical, chemical, or performance characteristics. In practical terms, such a material, component, element, or product may be effectively considered polyethylene-free.

The term "opacity", as used herein, is intended to refer to the degree to which materials resist transmission therethrough of rays of light. "Opacifiers" are therefore agents known in the art which are capable of imparting additional opacity to materials to which they are added, incorporated, or applied.

### THE ABSORBENT ARTICLE

The absorbent article (1) herein comprises a liquid permeable topsheet (2); an absorbent core (3); and a substantially liquid impermeable backsheet (4); wherein the absorbent core (3) is sandwiched between the said topsheet (2) and said backsheet (4); and wherein said backsheet is breathable. The backsheet (4) is made of a composite material, said composite material comprising, preferably consisting essentially of, even more preferably consisting of, a film and at least a layer of nonwoven fabric, wherein the film is positioned on a skin-facing surface of the backsheet and the nonwoven fabric is positioned on a garment-facing surface of the backsheet. The composite material being substantially free of an adhesive layer between said film and said nonwoven, preferably wherein the film and nonwoven are substantially co-formed.. Advantageously this allows to not only reduce the use of materials (such as adhesives) for improved sustainability but further allows to reduce the overall basis weight of the film and composite material so as to provide overall benefits towards scope 3 typer emissions. Moreover, a sensory experience for the user is provided for by the nonwoven surface which in absence of adhesive in state of the art lamination processes is able to convey a softer perception to the touch even at lower basis weights.

The absorbent articles herein are preferably selected from diapers, pants, briefs, liners, and towels.

Preferably, the film and nonwoven are co-formed, preferably extrusion co-formed, more preferably such that the nonwoven directly adjoins the film. By "directly" it is intended in absence of additional bonding layers like adhesives. Advantageously this permits the avoidance of adhesive coatings applied therebetween which allows to reduce overall basis weight of the material, CO2 emissions per piece and also potential risks of undesirable adhesive migration particularly when using lower basis weights of nonwovens.

An intermediate layer may be interposed between the film layer and the nonwoven layer. Good joining of the intermediate layer with the film and nonwoven layers results when the intermediate layer is bonded to the said layers both in molten state and thereafter when the structure has been solidified. The interlayer adhesion strength should be, at least, at the level of melt strength of the strongest layer; insufficient adhesion level could only give moderate improvements in draw-down and quickly produces interfacial instabilities by partial or total layer delamination caused by a significant difference in rheological behaviour between layers.

Preferably, the intermediate layer is obtained in one or more of the following ways: (i) The intermediate layer can be formed by chemical interaction between the film and nonwoven layers. In this embodiment, the polymers of the film and nonwoven may be selected to be mutually compatible, meaning that in a molten state and after solidification of the multilayer structure, they form a continuous phase together. Under extrusion temperatures, both the film polymers and the nonwoven polymers may form a blend matrix, thereby forming the intermediate layer during the extrusion process, particularly when they come into contact under heat at a common die; and/or (ii) The intermediate layer can be formed by chemical interaction between the film and nonwoven polymers, provided that the film layer and/or the nonwoven layer also contain a dispersed adhesive material (this being different from the use of adhesive as additional bonding layer as for example in traditional lamination and coating techniques). Under extrusion temperatures, the adhesive material may blend with the film polymer and/or the nonwoven polymer. The adhesive material dispersed in this manner may enhance compatibility between the film and nonwoven polymers. Depending on the degree of compatibility, the adhesive material can be added in an amount ranging from 0.5% to 10% by weight of the total polymer weight of the film and nonwoven layers, when the polymers tend to blend homogeneously in the molten state, or from 10% to 60% by weight when the polymers of the film and nonwoven layers do not blend homogeneously in the molten state.

In an embodiment, the co-formed film is a multi-layered structure and may comprise two layers: one composed of an extensional thinning behavior polymer, optionally containing a dispersed adhesive material, and another composed of an extensional thickening behavior polymer, optionally containing a dispersed adhesive material. Preferably, the same adhesive material can also be used for bonding the layered structure onto the nonwoven layer, thereby eliminating the need for a "distinct" adhesive material for bonding the film to the nonwoven.

The nonwoven fabric may comprise, preferably consists of, one or more layers; and wherein the nonwoven fabric has a basis weight from 5 to 50 gsm, preferably from 5 to 40 gsm, more preferably from 6 to 30 gsm, even more preferably from 7 to 20 gsm, most preferably from 8 to 10 gsm.

The film may have a basis weight of less than 10 gsm, preferably less than 8 gsm, more preferably from 1 gsm to 5 gsm, even more preferably from 1 gsm to 4 gsm, even more preferably from 2 gsm to 3 gsm, even more preferably about 3 gsm. Surprisingly, such forming process has been found to be particularly useful when further reducing the basis weight of the film materials. Advantageously, such low basis weight not only brings advantages with regards to less material usage and waste but further also brings a significant positive impact on scope 3 emissions reduction.

Preferably, the film (including all its layers in cases of a multilayered film) has a total thickness of less than 2.5 µm, preferably less than 2 µm, even more preferably from 0.5 µm to 1.5 µm. Especially in these embodiments with low film thickness (which allow for significantly reduced material usage and surprisingly found to, in combination with the nonwovens herein, provide for the desired mechanical performance) it is preferable that the film comprises an opacifier such that the opacity index of the film is at least 35%. In particular the opacity index may range from 40% to 100%, in particular from 45% to 95%, preferably from 50% to 85%, or from 65% to 80%. If the opacity is too low, the film material may appear too thin and provide undesirable see-though/translucency through which certain exudates could be viewable by a caregiver. If the opacity it too high, the amount of opacifier necessary may need to be increased to a level that would start to impact things such as mechanical performance of the film, especially when operating at extremely low basis weights.

For example, whiteness, brightness, and/or opacity may be provided to the film by the inclusion of opacifiers/colorants such as TiO2 (titanium dioxide), or sodium aluminum silicate, and optical brighteners on or in the material of the films themselves. Opacifiers used in the films according to the above embodiment preferably are not contained in an amount of more than 25%wt, preferably from 0.01%wt to 10%wt, by weight of the film composition.

The nonwoven may comprise fibers selected from the group consisting of polyolefin fibers, polyester fibers, and mixtures thereof; preferably wherein the nonwoven comprises fibers selected from polyethylene fibers, polypropylene fibers, polylactic acid fibers, and/or Polyethylene terephthalate fibers.

The nonwoven is preferably selected from the group consisting of spunbond, meltblown, and carded nonwovens, and mixtures thereof.

Preferably, the nonwoven comprises one or more spunbond nonwovens and/or meltblown nonwovens. Spunbond nonwovens provide excellent softness, flexibility, and drapability, contributing to a more sensory experience by-touch for the user while also offering good tensile strength and abrasion resistance. Meltblown nonwovens, with their fine fiber structure and dense network, enhance the mechanical integrity of the composite material by improving tear resistance, puncture strength, and barrier properties. These materials offer a balance of strength, flexibility, and processability, making them suitable for use in composite structures where both durability and lightweight properties are required.

More preferably, the nonwoven is a multilayer nonwoven comprising spunbond and meltblown nonwovens, such as spunbond-meltblown (SM), spunbond-meltblown-spunbond (SMS), spunbond-spunbond-meltblown-spunbond (SSMS), and the like. These multilayer structures leverage the complementary properties of spunbond and meltblown layers, resulting in improved mechanical integrity while maintaining a low basis weight and softness.

The film preferably comprises a copolymer of ethylene and methyl acrylate and preferably a thermoplastic polyester elastomer. A suitable copolymer for extrusion is for example commercially available Elvaloy AC1125 (a DOW-polymer ELVALOY^{™} AC 1125 Acrylate Copolymer manufactured and sold by Dow Inc.). A suitable thermoplastic polyester elastomer for extrusion is for example commercially available Hyrtel G5544 (manufactured and sold by the Celanese Corporation under its commercial brand Hytrel^{®}). Advantageously this allows for a reduced basis weight whilst at the same time retaining mechanical performance and resistance to tear.

Preferably, the film is essentially free of polyethylene. Without wishing to be bound by theory, the use of polyethylene, as is customary in state-of-the-art film formation for packaging films of absorbent articles and/or impermeable backsheets of absorbent articles, presents significant limitations, particularly in achieving substantial reductions in the basis weight of the resulting films below 10 gsm or even below 14 gsm while maintaining acceptable mechanical performance. Specifically, polyethylene-based films tend to exhibit inherent challenges in balancing reduced material usage with adequate tensile strength, puncture resistance, and flexibility. The absence of polyethylene in the film formulation allows for the incorporation of alternative polymeric compositions and processing techniques, such as coextrusion with elastomeric or semi-crystalline polymers, which can enhance film performance at lower basis weights.

The composite material may have a total basis weight of from 7 gsm to 30 gsm, preferably from 8 gsm to 25 gsm, even more preferably from 9 gsm to 20 gsm, even more preferably from 10 gsm to 15 gsm, even more preferably from 11 gsm to 14gsm. A lower basis weight contributes to material savings, reduced environmental footprint, and improved package flexibility, while still ensuring adequate barrier properties and mechanical integrity especially when in combination with significantly reduced film basis weight as described herein, a co-extruded nonwoven of the above indicated basis weight ranges cooperates synergistically in providing improved mechanical performance of the composite material.

In an embodiment, the articles herein further comprise two oppositely disposed elastic panels (5) and a fastening system, the fastening system comprising one or more male fasteners (6), preferably comprising hook-like fastening members; and wherein the nonwoven fabric of the composite material is the female fastener of the fastening system for cooperation with the male fastener(s); even more preferably wherein the article is free of additional landing zone for male fastener attachment. Advantageously such composite materials are particularly useful as landing zone replacement due to their resistance to delamination compared to traditionally adhesively bonded laminates, hence permitting good male fastener attachment and at the same further reducing the use of material and scope 3 emissions by elimination of a further component from the article. Indeed without wishing to be bound by theory, especially when reducing the basis weight of the nonwoven (e.g. to below 25gsm) delamination upon hook shear becomes a concern in traditional adhesive laminates.

In an embodiment that may be in alternative and/or addition to the above (e.g. non-refastenable and/or refastenable pants), the articles herein further comprise a front elastic belt (7) and a back elastic belt (8), the front and back belts being joined to each other at oppositely disposed side seams (9) to form a waist opening and two leg openings; and wherein each of the front and back belts comprise inner and outer nonwovens with an elastic material sandwiched therebetween, preferably the elastic material being selected from a perforated elastic film and elastic strands, and wherein the basis weight of the outer nonwovens is greater than the basis weight of the nonwoven fabric of the composite material. Advantageously this allows for a bulk differential that promotes softness in the waist region whilst cooperating to reduce the overall basis weight.

The article herein may further contain other features common in the art such as acquisition distribution layer(s) (10), lateral cuffs (11), transversal cuffs or pockets, elastic waistband(s) and the like.

Preferably, the film is breathable. The film may hence a water vapor transmission rate (WVTR) of at least 500 grams H2O/24-hour/m2, preferably at least 1 ,000 grams H2O/24-hour/m2, more preferably at least 2,000 grams H2O/24-hour/m2, even more preferably at least 3500 grams H2O/24-hour/m2, even more preferably of at least 4500 grams H2O/24-hour/m2, even more preferably of at least about 6,000 grams H2O/24-hour/m2, even more preferably of at least about 7,000 grams H2O/24-hour/m2, even more preferably of at least about 9,000 grams H2O/24-hour/m2, and/or preferably of from about 1,000 grams H2O/24-hour/m2 to about 10,000 grams H2O/24-hour/m2. Advantageously, this allows for breathability enhancement of the backsheet that reduces moisture build-up on the skin of the wearer whilst attaining acceptable mechanical performance.

The composite material is preferably substantially free of adhesive or adhesive polymer for providing adhesion between the film and the nonwoven fabric and is preferably free of laminates of film and nonwoven. The absence of adhesives and laminates reduces the risk of delamination over time, enhances recyclability, and maintains the mechanical properties of the composite material. Alternative adhesion methods, such as thermal bonding or mechanical interlocking, may be employed to achieve the necessary structural cohesion without compromising sustainability objectives.

### THE METHOD OF MANUFACTURE

A further aspect of the disclosure relates to method for manufacture of an absorbent article, the method comprising the, preferably sequential, steps of:
i. co-forming, preferably co-extruding, a film and nonwoven to form a composite material web;
ii. providing a liquid permeable nonwoven web;
iii. providing an absorbent core; and
iv. laminating the composite material web to at least one of the liquid permeable nonwoven web and the absorbent core such that the absorbent core is sandwiched therebetween.

The co-forming step can be selected from any one of the following: cast extrusion, blown film extrusion, extrusion-coating, extrusion-lamination, curtain coating extrusion, profile extrusion, filament spinning and/or spun-melt nonwoven extrusion; preferably cast extrusion, blown film extrusion, profile extrusion, filament spinning and/or spun-melt nonwoven extrusion.

Preferably, the composite material is substantially free of an adhesive layer between said film and said nonwoven, preferably wherein the composite material is substantially free of adhesive or adhesive polymer for providing adhesion between the at film and the nonwoven fabric; and/or is preferably free of laminates of film and nonwoven.

The absorbent article in the method herein is preferably according to any of the embodiments contained in the absorbent article section herein.

The article in the present method may comprise any of the features of the preferred embodiments described above, for example with regards to material composition of the film/nonwoven, basis weights etc.

### TEST METHODS

### Test Method: Opacity

Opacity by contrast ratio measurements are made using a 0°/45° spectrophotometer suitable for making standard Hunter L*a*b* color measurements (e.g. Hunterlab Labscan XE spectrophotometer, Hunter Associates Laboratory Inc., Reston Va. or equivalent). The diameter of the instrument's measurement port should be chosen such that only the region of interest is included within the measurement port. Analyses are performed in a room controlled at about 23° C.±2 C.° and 50%±2% relative humidity. Samples are conditioned at the same condition for 2 hours before testing.

Calibrate the instrument per the vender instructions using the standard black and white tiles provided by the vendor. Set the spectrophotometer to use the CIE XYZ color space, with a D65 standard illumination and 10° observer. Using cryogenic spray and scissors excise the topsheet specimen from the article for testing. Place the specimen flat against the instrument with the body facing surface toward the spectrophotometer's measurement port and the region of interest within the port. Place the white standard tile onto the opposing surface of the specimen such that it completely covers the measurement port. Take a reading for XYZ and record to 0.01 units. Without moving the specimen, remove the white plate and replace it with the black standard plate. Take a second reading for XYZ and record to 0.01 units. Repeat this procedure at a corresponding site for a total of ten (10) replicates specimens.

Opacity is calculated by dividing the Y value measured using the black tile as backing, divided by the Y value measured using the white tile as backing, then multiplying the ratio by 100. Record opacity to the nearest 0.01%. Calculate opacity for the 10 replicates and report the average opacity to the nearest 0.01%.

### EXAMPLES

### Example 1

### Base Polymer: Polypropylene

To produce a water vapour breathable polypropylene non-woven, a co-extruded multilayer film can be applied using extrusion-lamination, curtain application, or direct extrusion onto a polypropylene non-woven substrate. The multilayer film may comprise:

### 1. Extensional Thinning Layer:

This layer, optionally containing an adhesive material dispersed within it, is selected from a group of polymers demonstrating water vapour permeability values greater than 1 g mm/m² day, as measured by the ASTM E96B method. These polymers function based on absorption-desorption mechanisms (non-porous) and provide effective moisture management while maintaining film integrity. Suitable polymers include, but are not limited to:
Polyether-ester block co-polymer elastomers (e.g., Hytrel from Dupont or Arnitel from DSM), which offer flexibility and durability.

Styrene block co-polymers, known for their elasticity and resilience.

Polyamides 6 or 6.6, providing excellent mechanical strength and moisture resistance.

Polyethylene and Polybutylene terephthalate, offering a balance of flexibility and barrier properties.

Polyethylene oxide block co-polymers, which enhance moisture absorption and release. ABS (Acrylonitrile Butadiene Styrene), known for impact resistance and toughness.

Thermoplastic polyurethanes, valued for their elasticity and durability.

Polyether block amides (e.g., Pebax from Arkema), which combine elasticity with moisture management.

Bio-polymers like PLA (Polylactic Acid), offering sustainable and biodegradable alternatives.

Acrylic-co-polymers or their blends and co-polymers, providing enhanced bonding and elasticity.

The layer thickness typically ranges from 0.5 to 6 µm, optimizing breathability while maintaining structural integrity.

### 2. Extensional Thickening Layer:

This layer, optionally containing an adhesive material dispersed within it, contributes to enhanced mechanical strength and stability of the film. Suitable compositions include:
Low Density Polyethylene (LDPE) methyl or ethyl acrylate co-polymers or their anhydride or acid-modified variants, known for their flexibility and adhesive properties. Layer thickness ranges from 0.1 to 2 µm.

Acrylic or Methacrylic Acid Co-Polymers of Polyolefins, enhancing compatibility with various substrates. Layer thickness ranges from 0.1 to 2 µm.

lonomers, providing high impact strength and excellent barrier properties. Layer thickness ranges from 0.1 to 2 µm.

Ethylene Vinyl Acetate (EVA) Co-Polymers containing more than 18% vinyl acetate content, blended with a compatible tackifier at a mass proportion of 20-60% by weight. This blend offers improved flexibility and bonding performance. Layer thickness ranges from 0.1 to 2 µm.

This combination of polymers and layered configuration can achieve critical drawdown ratios higher than 150 and Mocon ASTM 1249 water vapour breathability values between 3,000 and 20,000 g/m² day, ensuring both high breathability and mechanical integrity.

### Example 2

### Bi-Component Core-Sheath Filament Extrusion for Enhanced Mechanical Integrity

Polyolefin filaments are extruded using bi-component core-sheath filament extrusion equipment or spun-melt nonwoven equipment. The composition includes:

### Filament Core:

Constitutes 10% to 90% by weight of the total filament section.

Made of Extensional Thickening Polyolefin, such as High Melt Strength Polypropylene or Low Density Polyethylene, providing enhanced mechanical integrity and draw stability. The polyolefin has an MFI of 2 to 30 and is blended with a hydrocarbon compatible tackifier (average molecular weight between 1,000 and 3,000) at 10% to 60% by weight, enhancing the bonding strength and flexibility.

### Filament Sheath:

Constitutes 10% to 90% by weight of the total filament section.

Made of Extensional Thinning Polyolefin, such as Polypropylene Homo-Polymer or Co-Polymer, or Linear Low Density Polyethylene, with an MFI of 2 to 30, ensuring smooth processability and optimized surface softness.

An alternative embodiment utilizes a single filament extrusion process, where a higher viscosity polymer is used at a lower volume percentage in the core compared to the polymer used in the sheath, achieving a self-structuring core-sheath filament configuration due to the differential viscosity and volume percentages.

### Example 3

### Blown Co-Extruded Multilayer Film for Improved Bubble Stability

In another embodiment, a blown co-extruded multilayer film is produced with enhanced bubble stability at high draw-down ratios. This configuration includes:

### Inner Layer:

Composed of an Extensional Thickening Layer such as Low Density Polyethylene, High Melt Strength Polypropylene, or in-situ branched linear polymers via crosslinking, accounting for 90% to 50% of the layer composition.

Blended with a Hydrocarbon Tackifier at 10% to 50%, ensuring improved mechanical strength and adhesion properties.

### Outer Layer:

Composed of an Extensional Thinning Layer such as Linear Low Density Polyethylene, Polypropylene Homo-Polymer, or Co-Polymers, providing excellent processability and optimized film softness.

This configuration achieves improved bubble stability and superior mechanical performance, making it particularly suitable for high-speed manufacturing processes.

### Example 4

### Breathable Back-Sheet for Diapers

Equipment: Extrusion laminating machine, 1.5 m width.

Substrate: Polypropylene homo-polymer spun-bond non-woven, 13 g/m².

### Layer Structure: A-B-Substrate

### A-Layer Composition:

Extensional thinning polymer: Polyether-Ester block co-polymer (Dupont Hytrel DYM350 NC010, MFI 15).

This layer provides high breathability and elasticity, ensuring comfort and flexibility.

### B-Layer (Tie Layer) Composition:

Extensional thickening polymer: Low Density Polyethylene-Ethylene Acrylate Co-Polymer (Dupont Bynel 22E804).

This layer enhances adhesion between the A-layer and the substrate while maintaining mechanical integrity.

### Process Settings at Stable Running:

Extrusion Temperature (Both Layers): 270°C
Die Gap (Hot): 0.4 mm
Vacuum Box and Cooling on Cast Roll Surface: Utilized for enhanced layer stability and uniformity.
Total Layer Thickness: 1.5 µm
A-Layer Thickness: 1 µm
B-Layer Thickness: 0.5 µm
Line Speed: 550 m/min
Draw Down Ratio: 133

### Performance Characteristic:

Breathability: Measured using Mocon Test ASTM 1249, the breathable back-sheet achieves a water vapour transmission rate of 5,200 g/m² day, ensuring effective moisture management and comfort.

This configuration provides an optimal balance of breathability and mechanical strength, suitable for high-performance diaper back-sheets.

### Example 5

### Breathable Back-Sheet for Diapers

Equipment: Extrusion laminating machine, 1.5 m width.

Substrate: Polypropylene homo-polymer spun-bond non-woven, 10 g/m².

### Layer Structure: A-B-Substrate

### A-Layer Composition:

Extensional thinning polymer: Polyamide 6 (Zytel ST7301 NC010).

This layer offers high breathability through moisture absorption-desorption mechanisms and provides durability and elasticity.

### B-Layer (Tie Layer) Composition:

Extensional thickening polymer: Low Density Polyethylene-Anhydride Modified Ethylene Acrylate Co-Polymer (Dupont Bynel 21E830).

This layer enhances adhesion to the substrate while maintaining flexibility and mechanical strength.

### Process Settings at Stable Running:

Extrusion Temperature (Both Layers): 265°C
Die Gap (Hot): 0.4 mm
Vacuum Box and Cooling on Cast Roll Surface: Utilized to stabilize the film structure and optimize layer uniformity.
Total Layer Thickness: 2.5 µm
A-Layer Thickness: 1.5 µm
B-Layer Thickness: 1 µm
Line Speed: 550 m/min
Draw Down Ratio: 160

### Performance Characteristic:

Breathability: Measured using Mocon Test ASTM 1249, this breathable back-sheet achieves a water vapour transmission rate of 4,200 g/m² day, ensuring effective moisture management and user comfort.

This configuration achieves a well-balanced breathable film with superior mechanical strength and elasticity, ideal for high-performance diaper back-sheets.

### Example 6

An absorbent article is made by laminating a liquid permeable topsheet to a backsheet being a composite of Examples 1, 2, 3, 4 or 5. An absorbent core being interposed between the topsheet and the backsheet.

## Claims

1. An absorbent article (1) comprising a liquid permeable topsheet (2); an absorbent core (3); and a substantially liquid impermeable backsheet (4); wherein the absorbent core (3) is sandwiched between the said topsheet (2) and said backsheet (4); and wherein said backsheet is breathable, **characterised in that** said backsheet (4) is made of a composite material, said composite material comprising, preferably consisting essentially of, even more preferably consisting of, a film and at least a layer of nonwoven fabric, wherein the film is positioned on a skin-facing surface of the backsheet and the nonwoven fabric is positioned on a garment-facing surface of the backsheet, and **in that** the composite material is substantially free of an adhesive layer between said film and said nonwoven, preferably wherein the film and nonwoven are substantially co-formed.

2. An article according to Claim 1, wherein the film and nonwoven are co-formed, preferably extrusion co-formed, such that the nonwoven directly adjoins the film.

3. An article according to any of the preceding Claims, wherein the nonwoven fabric comprises, preferably consists of, one or more layers; and wherein the nonwoven fabric has a basis weight from 5 to 50 gsm, preferably from 5 to 40 gsm, more preferably from 6 to 30 gsm, even more preferably from 7 to 20 gsm, most preferably from 8 to 10 gsm.

4. An article according to any one of the previous Claims, wherein the film has a basis weight of less than 10 gsm, preferably less than 8 gsm, more preferably from 1 gsm to 5 gsm, even more preferably from 2 gsm to 3 gsm.

5. An article according to any one of the previous Claims, wherein the nonwoven comprises fibers selected from the group consisting of polyolefin fibers, polyester fibers, and mixtures thereof; preferably wherein the nonwoven comprises fibers selected from polyethylene fibers, polypropylene fibers, polylactic acid fibers, and/or Polyethylene terephthalate fibers.

6. An article according to any one of the previous Claims, wherein the film comprises a copolymer of ethylene and methyl acrylate.

7. An article according to any one of the previous Claims, wherein the film is essentially free of polyethylene.

8. An article according to any one of the previous Claims, wherein the composite material has a total basis weight of from 7 gsm to 30 gsm, preferably from 8 gsm to 25 gsm, even more preferably from 9 gsm to 20 gsm, even more preferably from 10 gsm to 15 gsm, even more preferably from 11 gsm to 14gsm.

9. An article according to any of the preceding Claims, wherein the composite material is substantially free of adhesive or adhesive polymer for providing adhesion between the at film and the nonwoven fabric, and is preferably free of laminates of film and nonwoven.

10. An absorbent article according to any of the preceding Claims, further comprising two oppositely disposed elastic panels (5) and a fastening system, the fastening system comprising one or more male fasteners (6), preferably comprising hook-like fastening members; and wherein the nonwoven fabric of the composite material is the female fastener of the fastening system for cooperation with the male fastener(s); even more preferably wherein the article is free of additional landing zone for male fastener attachment.

11. An absorbent article according to any one of the preceding Claims, further comprising a front elastic belt (7) and a back elastic belt (8), the front and back belts being joined to each other at oppositely disposed side seams (9) to form a waist opening and two leg openings; and wherein each of the front and back belts comprise inner and outer nonwovens with an elastic material sandwiched therebetween, preferably the elastic material being selected from a perforated elastic film and elastic strands, and wherein the basis weight of the outer nonwovens is greater than the basis weight of the nonwoven fabric of the composite material.

12. A method for manufacture of an absorbent article, the method comprising the, preferably sequential, steps of:
i. co-forming, preferably co-extruding, a film and nonwoven to form a composite material web;
ii. providing a liquid permeable nonwoven web;
iii. providing an absorbent core; and
iv. laminating the composite material web to at least one of the liquid permeable nonwoven web and the absorbent core such that the absorbent core is sandwiched therebetween.

13. A method according to Claim 12, wherein the co-forming step is selected from any one of the following: cast extrusion, blown film extrusion, extrusion-coating, extrusion-lamination, curtain coating extrusion, profile extrusion, filament spinning and/or spun-melt nonwoven extrusion; preferably cast extrusion, blown film extrusion, profile extrusion, filament spinning and/or spun-melt nonwoven extrusion.

14. A method according to any one of Claims 12 to 13, wherein the absorbent article is according to any one of Claims 1 to 11.

15. A method according to any one of Claims 12 to 14, wherein the composite material is substantially free of an adhesive layer between said film and said nonwoven, preferably wherein the composite material is substantially free of adhesive or adhesive polymer for providing adhesion between the at film and the nonwoven fabric; and/or is preferably free of laminates of film and nonwoven.
